# EUROPEAN PATENT SPECIFICATION

(11) **EP 1 437 591 B1**
(45) Date of publication and mention of the grant of the patent: **10.06.2009**
(21) Application number: 02775280.7
(22) Date of filing: 01.10.2002
(51) Int. Cl.: G01N 33/493, G01N 33/70, G01N 33/68

(54) **METHOD FOR DIAGNOSING KIDNEY DISEASES**
METHODE ZUR DIAGNOSE VON NIERENERKRANKUNGEN
METHODE DE DIAGNOSTIC DE MALADIES RENALES

(30) Priority: 02.10.2001 JP 2001306445
(43) Date of publication of application: 14.07.2004
(73) Proprietor: Ikagaku Co., Ltd., Kyoto-shi, Kyoto 612-8486 (JP)
(72) Inventor: UCHIDA, Kazuo, c/o Ikagaku Co., Ltd., Kyoto-shi, Kyoto 612-8486 (JP)
(74) Representative: Kloiber, Thomas
(86) International application number: PCT/JP2002/010233
(87) International publication number: WO 2003/031967

(56) References cited:
- JP-A- 10 062 412
- JP-A- 11 064 333
- JP-A- 2000 193 662
- TIAN S ET AL: "Cystatin C measurement and its practical use in patients with various renal diseases" CLINICAL NEPHROLOGY, vol. 48, no. 2, 1997, pages 104-108, XP009063131 ISSN: 0301-0430
- IMANISHI M ET AL: "Glomerular hypertension as one cause of albuminuria in Type II diabetic patients" DIABETOLOGIA, vol. 42, no. 8, August 1999 (1999-08), pages 999-1005, XP002371671 ISSN: 0012-186X

## Description

### Field of the Art

The present invention relates to a method for diagnosing a kidney disease, and in particular to a method for reliably diagnosing the occurrence of a glomerular lesion (glomerular hypertension status) and tubulointerstitial disorder in patients with diabetes by performing combined measurement of cystatin C and creatinine in urine as measured subjects.

### Background Art

Kidney diseases are classified into glomerular origin and interstitial renal tubular origin by a primary focus site of a lesion. Conventionally, various glomerular lesions have been diagnosed at a point in time when persistent proteinuria (posivive in a test paper method or persistent protein of 500 mg or more/day in urine) emerged.

In recent years, it has become possible to measure various proteins discharged into urine at a trace amount. In particular, it has been demonstrated that an increase in a non-discharged amount of albumin or transferrin in urine, i.e., the trace amount of albumin or transferrinuria precedes persistent proteinuria. Thus, it is generalized that nephropathy is diagnosed by the emergence of a trace amount of albumin or transferrinuria. Also, recently, for example, as the classification of disease stages for diabetic nephropathy, the first stage (nephropathy prophese), the second stage (early phase of nephropathy), the third stage (apparent nephropathy phase), the fourth stage (renal failure phase) and the fifth stage (dialytic treatment phase) have become common. According to this classification, the stage at which the trace amount of albumin or the transferrinuria is exhibited is classified into an early phase of the nephropathy stage.

However, pathologically, mild to moderate erosive lesions are already present and the presence of nodular lesions is known at this early phase of the nephropathy stage. Therefore, this stage is not necessarily an early phase of nephropathy, and is commonly understood as a phase capable of being diagnosed by current clinical examinations. Thus, it has been anticipated to develop test methods capable of detecting abnormalities which occur at an earlier stage (first stage) than the trace amount of albumin or transferrinuria.

Thus, the present inventor has found that infiltration of leukocytes (particularly, neutrophils and monocytes/macrophages) into glomerulus becomes a starting point of proteinuria emergence in glomerular nephritis including diabetic nephropathy, and has already filed the technology for measuring urinary lactoferrin and myeloperoxidase as a method for detecting that neutrophils infiltrates into glomerulus (Japanese Published Unexamined Patent Application No. H9-72906). As a result of further study, the present inventor has found that it is useful for early diagnosis of glomerular nephritis to detect the infiltration of neutrophils into glomerulus, and has already filed an application (Japanese Published Unexamined Patent Application No. H9-274036).

In this method, urinary lactoferrin and myeloperoxidase are simultaneously measured and the difference (Lf-MPO) thereof is calculated as lactoferrin derived from glomerulus to make it an early diagnostic index of nephropathy. However, problems in this method include particularly that the urinary lactoferrin value is influenced by factors other than nephropathy in addition to the necessity to simultaneously measure two components. That is, lactoferrin is present in seminal plasma at a high concentration in males, and thus it has shown that a false positive is given when seminal fluid is contaminated in urine in males during their reproductive age or higher (from fifth grade primary school students and above).

Thus, the present inventor has found a related event having the same meaning as measuring urinary lactoferrin and myeloperoxidase as the method for detecting that inflammatory cells (neutrophils, monocytes/macrophages) infiltrate into glomerulus and as a method useful for early diagnosis of nephropathy, has developed a method for measuring concentrations of urinary cystatin C and has filed an application (Japanese Published Unexamined Patent Application No. H11-64333). According to a subsequent study, it was found as an important factor in renal disorder progress that glomerular hypertension is present upstream as a phenomenon where neutrophils and macrophages infiltrate into glomerulus.

Subsequently, glomerular hypertension is illustrated. The most important function of glomerulus is the filtration function, and blood at about 100 ml per minute and about 14 L per day is filtrated. This glomerular filtration requires pressure, and about 50 mmHg of hydrostatic pressure acts on the glomerular capillary. This intraglomerular pressure is referred to as glomerular blood pressure, and the elevation thereof is termed as glomerular hypertension. The glomerular blood pressure is regulated by systemic blood pressure and a ratio of afferent arteriole resistance and efferent arteriole resistance, and higher pressure than those in other capillary vessels is retained by this structure to enable filtration of a large amount of blood in the glomerulus. The glomerular hypertension indicates an increase of a glomerular filtration rate (GFR), i.e., increased renal function.

Next, causes of glomerular hypertension are illustrated. In diabetes, the increase of GFR (glomerular excessive filtration) is observed as the earliest change in the kidney, and glomerular hypertension is described as one of the causes. In diabetes, dilatation of the afferent arteriole is a major factor of glomerular hypertension. It is believed that dilatation factors are a contraction disorder of smooth muscle per se which composes the blood vessel and increase of vasodepressor materials. Candidates of vasodepressor materials include atrial natriuretic peptide (ANP), carbon monoxide (NO), vasodepressor prostaglandin and the like. Glomerular hypertension is also observed in glomerular nephritis when inner pressure of residual glomerulus is elevated along with advancement of a partial lesion of the glomerulus.

Next, the relation of glomerular hypertension and renal disorder is illustrated. Persistent elevation of glomerular blood pressure elicits endothelial cell disorder in the glomerulus and induces infiltration of platelets and macrophages. Various growth factors are released from these infiltrated cells, and these growth factors act on mesangial cells to increase extracellular matrix resulting in glomerulosclerosis. This is referred to as the glomerular hypertension hypothesis (Hostetter, TH et al., Am. J. Med., 72:375-380, 1982), and indicates that glomerular hypertension is a risk factor in advancement of progressive glomerulosclerosis and renal failure. Thus, as a countermeasure for glomerular hypertension, it is necessary to reduce glomerular blood pressure as well as systemic blood pressure. Therefore, hypotensive drugs which dilate the efferent arteriole along with the afferent arteriole (angiotensin converting enzyme inhibitor, angiotensin 2 receptor antagonist, long-acting Ca antagonist) are described as being effective.

Next, the method for measuring glomerular blood pressure is illustrated. It is possible to measure glomerular blood pressure by a micropuncture method in experimental animals. Since this method cannot be applied to humans, the glomerular blood pressure cannot be directly measured. However, Kimura et al., have designed a method where glomerular blood pressure and afferent and efferent arteriole resistances are calculated by combining a pressure-diuresis curve, a serum protein concentration, renal clearance tests (measurement of GFR and RPF) and the like (Kimura, G. et al., Circulation Research 69:421-428, 1991).

From the above context, the measurement of glomerular blood pressure seems to be important as a method for early diagnosis of kidney disease including diabetic nephropathy and for monitoring drug efficacy upon administering a hypotensive drug. However, to know estimates of glomerular blood pressure, clinical examination relies on a method in which GFR is obtained by endogenous creatinine clearance or a method by inference from an excretion amount of trace amounts of proteins of which the majority is albumin into urine. However, the present inventor has discovered that the urinary excretion dynamic state of cystatin C (Low molecular protein with a molecular weight of 13,359 kDa and an isoelectric point of 9.3, of which concentration in blood is about 700 µg/L. Approximately 99% of it is filtrated at the glomerulus, and most of all is reabsorbed at renal tubule.) exhibits a favorable relation with creatinine which is freely filtrated at the glomerulus, is not reabsorbed at the renal tubule and is discharged into the urine, and has confirmed that the concentration of cystatin C in urine is a detectable amount, and has already filed an application as an early diagnostic method for nephropathy and nephritis (Japanese Published Unexamined Patent Application No. H11-64333).

However, it has been recently shown that it is important to establish a method for early and reliable diagnosis of a tubulointerstitial lesion which is another major lesion site of kidney diseases. The existence of a renal tubular abnormality in diabetes has been known since the report of Almani-Ebstein. Thereafter, interest in the renal tubule has increased in connection with the progress of studies on the effects of insulin on the renal tubular reabsorption mechanism (Saudek, C. D. et al., Diabetes 23:240-246, 1974; DeFronzo, R. A. et al., J. Clin. Invest., 55:845-854, 1975) and chemical analysis of renal tubular basement membrane in diabetes (Sato, T., Tonyobyo 8:303, 1975). Moreover, an abnormal metabolism or vascular disorder at a level of renal tubular epithelial cells are conceivable as causes of abnormal function in the renal tubule in diabetes.

Conventionally, β2-microglobulin, lysozyme, free right chain, retinol binding protein and α-microglobulin have been used as indices of renal tubular proteinuria observed during renal tubular disorder. Moreover, various enzymes are present in the urine and parts of the enzymes are derived from the renal tubule. Thus, activity of these urinary enzymes is increased by the renal tubular disorder, and become indices of the renal tubular disorder. The enzymes derived from the renal tubule are broadly divided into two. The first one is an enzyme present in lysosomes in proximal renal tubular epithelial cells and glomerular epithelial cells, N-acetyl-β-D-glucosaminidase (NAG). The second are enzymes present at the brush border of the proximal renal tubule, alkaline phosphatase (ALP), alanine aminopeptidase (AAP) and the like (Kaizu, K., Japan Medical Journal No. 3537, 139, 1992). Subsequently, with respect to the abnormal renal tubule in a patient group with diabetes, the elevation of urinary enzyme activity has been observed even in patients with negative urinary proteins (Kaizu, K., Japan Medical Journal, No. 3537, 139, 1992), and cases of increased β2-microglobulin discharged into the urine, which is a urinary low molecular protein has been observed (Kikkawa, R. et al., Tonyobyo 22:621-629, 1979). In this way, it has been shown that abnormal functions of the renal tubule are present regardless of the presence or absence of glomerular disorder in patients with diabetes.

Therefore, it is required to examine the presence or absence of the renal tubular disorder as well as to detect the glomerular lesion (glomerular hypertension status which is the first symptom) for early diagnosis of diabetic nephropathy. On the other hand, it has been shown that the tubulointerstitial lesion is a common change observed in progressive kidney diseases and that the degree of the tubulointerstitial lesion is more significantly correlated with renal function than that of the glomerular lesion. That is, it has been shown that an increase of glomerular permeability and ischemia elicit renal tubular disorder in not only primary interstitial nephritis but also glomerular diseases and vascular disorders, and that the tubulointerstitial lesions such as infiltration of inflammatory cells, interstitial fibrosing and renal tubular atrophy are formed at interstitium by factors produced from renal tubular epithelial cells which undergo disorders (Yamasaki, K., Journal of Clinical and Experimental Medicine (Igaku No Ayumi) 190:62-67, 1999). The cause of the tubulointerstitial disorder involved in the glomerular lesion includes urinary proteins as a particularly clinically important factor. It has been shown that the urinary protein is a mediator of renal failure progress as the cause of tubulointerstitial disorder along with proteinuria. It has been shown that infectious diseases, immunological factors, drugs, heavy metals, urinary tract occlusion, reflux, ischemia and the like are involved in other factors which make the tubulointerstitial disorder progress (Endo, M. et al., Mebio 18:55-60, 2001).

At present, measurement of the above urinary low molecular protein and urinary enzymes derived from the renal tubule is frequently used as the examination method for evaluating the renal tubular function. However, disease detection sensitivity of these examination methods is not sufficient, and it is required to simultaneously measure blood glucose levels, HbA1c and urinary albumin and to carefully observe the clinical course in early diagnosis of diabetic nephropathy (Kimura, K. et al., Tonyobyo 29: 995-1000, 1986). Meanwhile, it cannot be said that evaluation of the above examination method by measuring urinary low molecular proteins and urinary enzymes is sharp also in the early diagnosis of the tubulointerstitial disorder involved in the glomerular lesion.

The document JP 2000 -- 193662 refers to a latex agglutination immunoassay kit for the detection of cystatin C comprising anti-human cystatin C antibodies. The known method for diagnosing renal dysfunction comprises measuring cystatin C in urine samples for the purpose of diagnosing nephric tubule damage at an early stage.

The document JP 11-064.333 also discloses a diagnostic kits containing a reagent comprising anti-human cystatin C antibodies. This document further shows a correlation between the excretion of cystatin C and creatinine in urine to be a marker for nephropathy.

The publication TIAN S. et al.: "Cystatin C measurement and its practical use in patients with various renal diseases" CLINICAL NEPHROLOGY, vol. 48, no. 2, 1997, pages 104-108, refers to a method for diagnosing kidney diseases were in cystatin C values are measured in serum and urine as an indicator for renal disease using a commercially available ELISA kit. The publication discloses that cystatin C levels can be used to estimate the glomerular filtration rate and renal tubular damage.

The present invention has been made by focusing on these problems, and is intended to provide a method for reliably diagnosing kidney diseases by a novel examination method in place of the conventional method, and among others, to provide a method for diagnosing diabetic nephropathy at an early stage regardless of whether or not glomerular hypertension is preliminary to a tubulointerstitial disorder and a method for diagnosing the occurrence of tubulointerstitial disorder following glomerular lesion and primary tubulointerstitial disorder at an early stage.

### Brief Summary of the Invention

The method for diagnosis of the present invention is characterized by the features of claim 1. In particular the kidney disease is diagnosed by measuring a cystatin C concentration and a creatinine concentration discharged in urine of a patient with diabetes. That is, according to the present invention, it is possible to screen the presence or absence of the kidney disease by measuring the cystatin C concentration and the creatinine concentration discharged in urine. More preferably, it is possible to screen the presence or absence of the kidney disease by measuring the cystatin C concentration and the creatinine concentration discharged in urine.

Also, the method for diagnosis of the present invention is characterized in that the kidney disease is screened by a measured value per se of the cystatin C concentration or by calculating a ratio of the cystatin C concentration to the creatinine concentration and using it.

The above clearance substance which is subject to the measurement is preferably one or more of any of creatinine, inulin or sodium thiosulfate, and is more preferably creatinine.

It is preferable to use immunological assay methods such as an enzyme immunoassay, a latex agglutination assay and an immunochromatograpy method for the above measurement of the cystatin C concentration and to use a chemical method or an enzymatic method for the measurement of the clearance substance concentration.

Also, the method for diagnosis of the present invention is characterized in that the kidney disease is diagnosed by measuring the cystatin C concentration and the creatinine concentration in urine and the method comprises at least an anti-human cystatin C antibody and a reagent for the measurement of creatinine concentration by a chemical or enzymatic method.

Also, the method for diagnosis of the present invention is characterized by measuring the urinary cystatin C concentration and the creatinine concentration using urine in a large number of normal subjects, and using a cut-off value of the cystatin C concentration and a cut off value of a ratio of the cystatin C concentration to the creatinine concentration calculated from these measurement values.

Also, the method for diagnosis of the present invention is used for diagnosing the presence or absence of a renal tubular disorder in patients with diabetes by measuring the cystatin C concentration to the creatinine concentration discharged into the urine (UCCR) in a large number of normal subjects and making a cut-off value calculated from these measurement values as a standard, and comprises at least an anti-human cystatin C antibody for immunologically assaying the cystatin C concentration as described above.

Also, the method for diagnosis of the present invention is a method for diagnosing the presence or absence of a renal tubular disorder in patients with diabetes by measuring the cystatin C concentration to the creatinine concentration discharged into the urine (UCCR) in a large number of normal subjects and making a cut-off value calculated from these measurement values as a standard, then being used for diagnosing the presence or absence of glomerular hypertension in the above patients with diabetes in whom no renal tubular disorder has been observed by making a cut-off value calculated from the measurement values of the concentration of cystatin C discharged in the urine in the above large number of normal subjects as a standard, and comprises at least an anti-human cystatin C antibody for immunologically assaying the cystatin C concentration as described above.

Also, the method for diagnosis of the present invention is a method for diagnosing the presence or absence of renal tubular disorder in patients with diabetes by measuring the cystatin C concentration to the creatinine concentration discharged into the urine (UCCR) in a large number of normal subjects and making a cut-off value calculated from these measurement values as a standard, then being used for diagnosing the presence or absence of glomerular hypertension in the above patients with diabetes in whom a renal tubular disorder has been observed by making a cut-off value calculated from the measurement values of the concentration of creatinine discharged in the urine in the above large number of normal subjects as a standard, and comprises at least an anti-human cystatin C antibody for immunologically assaying the cystatin C concentration as described above.

### Brief Description of the Drawings

Fig. 1 is a drawing showing the correlation of urinary cystatin C concentrations and creatinine concentrations in protein negative and positive groups.
Fig. 2 is a flowchart for showing a procedure for early identification of diabetic nephropathy.
Fig. 3 is a drawing showing a cut-off value calculation example of urinary cystatin C concentrations and a cut-off value calculation example of urinary cystatin C/creatinine ratios.
Fig. 4 is a drawing showing actual examples of cystatin C/creatinine ratios in the urine of patients proteinuria positive and patients with urinary tract infection.
Fig. 5 is a drawing showing actual examples of a cystatin C/creatinine ratio measurement in the urine of patients with nephrosis syndrome and tubulointerstitial nephritis.
Fig. 6 is a drawing showing a detection sensitivity comparison of tubulointerstitial lesion involved in urinary tract infection.
Fig. 7 is a drawing showing calculation examples of cut-off values of cystatin C concentrations, creatinine concentrations and cystatin C/creatinine ratios (UCCR) using urine of healthy person.
Fig. 8 is a drawing showing the correlation of urinary cystatin C concentrations and creatinine concentrations in patients with diabetes by dividing into two groups with UCCR more than and less than a cut-off value (0.7).
Fig. 9 is a drawing diagnosing the presence or absence of glomerular hypertension in patients with diabetes by dividing into two groups with UCCR more than and less than a cut-off value (0.7).
Fig. 10 is a drawing comparing usefulness of the method of the present invention with that of the conventional method with respect to patients with diabetes having complications from urinary tract infection.
Fig. 11 is a drawing comparing usefulness of the method of the present invention with that of the conventional method with respect to patients with diabetes having complications from urinary tract infection.
Fig. 12 is a drawing showing urinary dynamics of cystatin C and creatinine in patients exhibiting proteinuria.
Fig. 13 is a drawing comparing UCCR values in a proteinuria positive patient group with those in a healthy group (control).
Fig. 14 is a drawing showing a diagnostic procedure of a tubulointerstitial disorder following a glomerular lesion.

### Detailed Description of the Invention

The kit for diagnosing kidney disease according to the present invention makes it the gist to diagnose the kidney disease by making urine discharged from a patient a specimen and quantitatively measuring a cystatin C concentration and a clearance substance concentration present in this specimen.

Here, it is preferable to use, for example, an immunoassay means such as an enzyme immunoassay method, a latex agglutination method, an immunoluminescence analysis method and an immunochromatography method known in the art for measurement of cystatin C concentration. In the present invention, among the above immunoassay means, the enzyme immunoassay method is especially preferably used in terms of good measuring sensitivity and ease for making into kits.

Also, in the present invention, as an antibody for detecting the urinary cystatin C, it is possible to use any of a polyclonal or monoclonal antibody so long as it is an antibody which reacts specifically with cystatin C. Specifically, when using the polyclonal antibody, an anti-human cystatin C polyclonal antibody can be used, and when using the monoclonal antibody, anti-human cystatin C monoclonal antibody can be used.

Also, in the present invention, to enhance the detection sensitivity of cystatin C, it is possible to use two types of anti-human cystatin C antibodies which have different reaction sites to cystatin C as the antibodies for detecting cystatin C. That is, one antibody is rendered the first antibody and another is rendered the second antibody. The detection sensitivity of cystatin C can be enhanced by firstly reacting the first antibody with cystatin C, and then reacting the second antibody with a complex formed by this reaction to form a complex made up of the first antibody, cystatin C and the second antibody. For the above first and second antibodies, it is possible to use any of a polyclonal or monoclonal antibody so long as it is an antibody which reacts specifically with cystatin C.

Furthermore, when the enzyme immunoassay method is used as the immunoassay means, it is possible to use a labeled enzyme by binding to the above second antibody in the method known in the art.

The immunoassay means which can be used in the present invention is not especially limited to the above means. When a sandwich ELISA method in which urine discharged from a patient is used as a specimen and the above first and second antibodies are used is employed as a specific example means, an outline of the detection method of cystatin C in urine is illustrated as follows. That is, it is characterized in that cystatin C is bound to the first antibody immobilized on a carrier, subsequently the second antibody binding a labeled enzyme is bound to this first antibody binding cystatin C, and a binding amount of the second antibody is measured by an enzyme-substrate reaction to quantify a concentration of cystatin C in the specimen. Such a sandwich ELISA method can be preferably used in the present invention because detection sensitivity is high and detection speed is fast which takes several hours.

When the second antibody binding the labeled enzyme is used as the above, the obtained data are represented by absorbance, only by which the concentration of cystatin C cannot be calculated. Thus, the concentration of cystatin C present in the specimen can be quantitatively calculated by separately preparing standard solutions of cystatin C at different concentrations, and making a standard curve on which an absorbance is plotted for the concentration of cystatin C.

Also, in the present invention, the clearance substance subject to measurement can be appropriately selected from the above-mentioned creatinine, inulin and sodium thiosulfate. When creatinine is selected as the clearance substance, a quantification method of creatinine concentrations is not especially limited, and is employed from methods known in the art such as chemical methods such as a reaction developing an umber color in an alkali picric acid solution, a reaction developing a pale magenta color in alkali 3,5-dinitrobenzoic acid and a reaction developing a red color by alkali sodium nitroprusside, and an enzymatic method using a creatinine degrading enzyme in soil bacteria, and the like. Also, the quantification methods of inulin and sodium thiosulfate can be employed from the methods known in the art, and are not especially limited.

The present invention is characterized in that urine discharged from a patient is used as a specimen as the above, a ratio of a cystatin C concentration to a clearance substance concentration obtained from this is calculated, and kidney disease is diagnosed using this. Specifically, the presence or absence of renal tubular disorder is diagnosed by whether or not the ratio of cystatin C concentration to clearance substance concentration (hereinafter, referred to as "cystatin C/clearance substance ratio" if necessary) of a certain patient exhibits a higher value compared with a cut-off value of the cystatin C/clearance substance ratio previously obtained in a normal group.

That is, in the present invention, it is possible to diagnose the presence or absence of renal tubular disorder in the kidney disease by measuring the cystatin C/clearance substance ratio discharged in urine in a large number of normal subjects and making the cut-off value calculated from these measurements a standard. Here, the cystatin C/clearance substance ratio is an index for diagnosing the presence or absence of renal tubular disorder in kidney disease.

The cut-off value is a value corresponding to an upper limit value obtained by making a histogram on the basis of data of the cystatin C/clearance substance ratio for a large number of normal subjects and statistically processing it, and a standard value for determining the presence or absence of a renal tubular disorder in the kidney disease. Here, for statistical processing, it is possible to appropriately employ a parametric method where parameters (mean value, standard deviation) are calculated from selected specimens depending on a distribution of the data and a value corresponding to the upper limit value (95%) is calculated from these values, or a non-parametric method where a curve is approximated to a histogram of selected specimens and a value corresponding to the upper limit value (97.5%) is calculated.

And, when the cystatin C/clearance substance ratio in urine of a patient exceeds the cut-off value of the cystatin C/clearance substance ratio in urine of the above normal subject group, the patient is diagnosed to exhibit renal tubular disorder in the kidney disease. On the contrary, when the cystatin C/clearance substance ratio in urine of a patient is below the cut-off value of the cystatin C/clearance substance ratio in urine of the above normal subject group, the patient is diagnosed as not exhibiting renal tubular disorder in the kidney disease.

For example, when creatinine is selected as the clearance substance, the presence or absence of renal tubular disorder in the kidney disease is diagnosed as follows. That is, it is possible to diagnose the presence or absence of renal tubular disorder in the kidney disease by measuring the cystatin C/creatinine ratio discharged in urine in a large number of normal subjects and making the cut-off value calculated from these measurements a standard.

And, when the cystatin C/creatinine ratio in urine of a patient exceeds the cut-off value of the cystatin C/creatinine ratio in urine of the above normal subject group, the patient is diagnosed to exhibit renal tubular disorder in the kidney disease. On the contrary, when the cystatin C/creatinine ratio in urine of a patient is below the cut-off value of the cystatin C/creatinine ratio in urine of the above normal subject group, the patient is diagnosed not to exhibit renal tubular disorder in the kidney disease.

In the present invention, for a patient who is judged not to exhibit renal tubular disorder when the presence or absence of renal tubular disorder in the kidney disease is diagnosed using the above cystatin C/creatinine ratio as the index, it is possible to further diagnose the presence or absence of glomerular hypertension in the kidney disease using the cystatin C concentration as the index. That is, the presence or absence of glomerular hypertension in the kidney disease is diagnosed by whether or not the cystatin C concentration of the patient shows a significantly higher value compared with the cut-off value of cystatin C concentration in normal subjects previously obtained.

That is, in the present invention, it is possible to diagnose the presence or absence of glomerular hypertension in the kidney disease for a patient in whom renal tubular disorder in the kidney disease has not been observed by measuring the concentration of cystatin C discharged in urine in a large number of normal subjects and making the cut-off value calculated from these measurements the standard. Here, the concentration of cystatin C is an index to diagnose the presence or absence of glomerular hypertension in the kidney disease for a patient in whom renal tubular disorder in the kidney disease has not been observed.

Also, the cut-off value is a value corresponding to an upper limit value obtained by making a histogram on the basis of data of the cystatin C concentration for a large number of normal subjects and statistically processing it, and a standard value for determining the presence or absence of glomerular hypertension in the kidney disease. Furthermore, for the cut-off value, it is possible to appropriately employ the parametric or non-parametric method depending on data distribution aforementioned.

And, when the cystatin C concentration in urine of a patient exceeds the cut-off value of the cystatin C concentration in urine of the above normal subject group, the patient is diagnosed to exhibit glomerular hypertension in the kidney disease. On the contrary, when the cystatin C concentration in urine of a patient is below the cut-off value of the cystatin C concentration in urine of the above normal subject group, the patient is diagnosed not to exhibit glomerular hypertension in the kidney disease.

Also, in the present invention, for a patient who is judged to exhibit renal tubular disorder when the presence or absence of renal tubular disorder in the kidney disease is diagnosed using the above cystatin C/creatinine ratio as the index, it is possible to further diagnose the presence or absence of glomerular hypertension in the kidney disease using the clearance substance concentration as the index. That is, the presence or absence of glomerular hypertension in the kidney disease is diagnosed by whether or not the clearance substance concentration of the patient shows a significantly higher value compared with the cut-off value of clearance substance concentration in the normal subjects previously obtained.

That is, in the present invention, it is possible to diagnose the presence or absence of glomerular hypertension in the kidney disease for a patient in whom renal tubular disorder in the kidney disease has been observed by measuring the concentration of clearance substance discharged in urine in a large number of normal subjects and making the cut-off value calculated from these measurements the standard. Here, the concentration of clearance substance is an index to diagnose the presence or absence of glomerular hypertension in the kidney disease for the patient in whom renal tubular disorder in the kidney disease has been observed.

The cut-off value is a value corresponding to an upper limit value obtained by making a histogram on the basis of data of the clearance substance concentration for a large number of normal subjects and statistically processing it, and a standard value for determining the presence or absence of glomerular hypertension in the kidney disease. In addition, for the cut-off value, it is possible to appropriately employ the parametric or non-parametric method depending on the data distribution aforementioned.

And, when the clearance substance concentration in urine of a patient exceeds the cut-off value of the clearance substance concentration in urine of the above normal subject group, the patient is diagnosed to exhibit glomerular hypertension in the kidney disease. On the contrary, when the clearance substance concentration in urine of a patient is below the cut-off value of the clearance substance concentration in urine of the above normal subject group, the patient is diagnosed as not exhibiting glomerular hypertension in the kidney disease.

For example, when creatinine is selected as the clearance substance, the presence or absence of glomerular hypertension in the kidney disease is diagnosed as follows. That is, it is possible to diagnose the presence or absence of glomerular hypertension in the kidney disease by measuring the creatinine concentration discharged in urine in a large number of normal subjects and making the cut-off value calculated from these measurements a standard.

And, when the creatinine concentration in urine of a patient exceeds the cut-off value of the creatinine concentration in urine of the above normal subject group, the patient is diagnosed to exhibit glomerular hypertension in the kidney disease. On the contrary, when the creatinine concentration in urine of a patient is below the cut-off value of the creatinine concentration in urine of the above normal subject group, the patient is diagnosed as not exhibiting glomerular hypertension in the kidney disease.

As illustrated above, in the present invention, first it is possible to reliably diagnose the presence or absence of renal tubular disorder in the kidney disease by making the cystatin C/clearance substance ratio the index. Also, in the present invention, it is possible to reliably diagnose the presence or absence of glomerular hypertension by making the cystatin C concentration the index for a patient in whom renal tubular disorder has not been observed in the above diagnosis. Further, in the present invention, it is possible to reliably diagnose the presence or absence of glomerular hypertension by making the clearance substance concentration the index for a patient in whom renal tubular disorder has been observed in the above diagnosis.

In order to effectively perform the above diagnosis of the kidney disease, it is preferred that the kit for diagnosis of the present invention at least comprises an immunoassay means for detecting the aforementioned cystatin C. Specifically, it is possible to employ a means known in the art such as an enzyme immunoassay method, a latex agglutination method, an immunoluminescence analysis method and an immunochromatography method. In the present invention, in the above immunoassay means, the enzyme immunoassay method is especially preferably used in terms of good measuring sensitivity and ease in making into kits.

Also, in the present invention, as a antibody for detecting urinary cystatin C, it is possible to use any of a polyclonal or monoclonal antibody so long as it is an antibody which reacts specifically with cystatin C. Specifically, when using the polyclonal antibody, an anti-human cystatin C polyclonal antibody can be used, and when using the monoclonal antibody, an anti-human cystatin C monoclonal antibody can be used.

And, in the present invention of a kit for diagnosis, such antibodies are preferably used in a reagent form or in an immobilized form onto a carrier such as a microplate.

In the present invention, when a sandwich ELISA method is employed as the immunoassay means, it is preferable to use two types of anti-human cystatin C antibodies which have different reaction sites to cystatin C, and to render one antibody the first antibody and another the second antibody.

And, in the present invention of a kit for diagnosis, the above first antibody is preferably used in an immobilized form onto a carrier such as a microplate, and the above second antibody in a reagent form.

In addition to the aforementioned immunoassay means, the kit for diagnosis of the present invention comprises standard solutions for quantifying the concentrations of cystatin C. By comprising such standard solutions, labor to separately prepare the standard solutions of cystatin C is saved. Thus, it is possible to easily make a standard curve on which an absorbance is plotted to the concentration of cystatin C and quantitatively calculate the concentration of cystatin C present in the specimen.

Also, in the present invention, it is possible to comprise a reagent for measuring the concentration of clearance substance by the chemical or enzymatic method in addition to the aforementioned immunoassay means or the standard solutions for quantifying the concentration of cystatin C. Such reagents are not especially limited so long as they are usually used in the above chemical or enzymatic method known in the art. For example, when a Jaffe reaction developing an orange red color in an alkali picric acid solution is utilized in the case where creatinine is selected as the clearance substance, it is preferable to comprise one or more reagents selected from a deproteinizing reagent, a picric acid reagent and a sodium hydroxide solution.

The present inventor carefully observing excretion dynamics of cystatin C into urine and consequently discovering the fact that cystatin C is discharged into urine retaining a good correlation with creatinine which is a clearance substance regardless of being urinary protein positive or negative, the present invention has been completed.

The relevance of this discovered novel phenomenon with diagnostic methods of the kidney disease is illustrated below. Cystatin C is a low molecular protein with a molecular weight of 13, 000 and the protein with an isoelectric point of 9.3 which is the strongest positive charge among proteins present in urine. A gene and promoters thereof of cystatin C are housekeeping types, and cystatin C is always produced and secreted from nucleated cells throughout the body at a constant rate without being affected by environmental variations which occur outside or inside of cells. Therefore, the concentration in serum is constant from infants to adults without being affected by prerenal effects, cystatin C is a free type without forming a complex with other components in serum, and thus cystatin C is suitable for physical property as a marker of GFR.

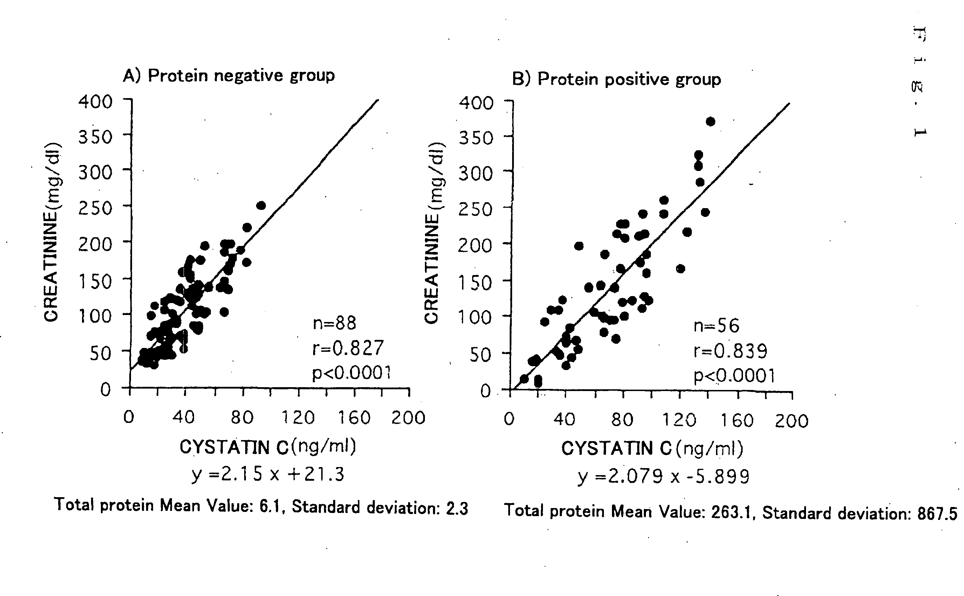

The present invention has been made by focusing on the discovery that this basic low molecular protein, cystatin C and creatinine which is the marker of endogenous GFR which is unabsorbed at the renal tubule are discharged retaining a good correlation under a condition where the tubulointerstitial function is normal, regardless of the presence or absence of urinary proteins. Fig. 1 shows a correlation of the urinary cystatin C concentration with the creatinine concentration in a urinary protein negative group and positive group. It is believed that due to being processed first and foremost by a protein reabsorption mechanism at the proximal renal tubule, i.e., acidification of endocytosis-lysosomeproteolytic system by reason of its characteristic of strongly- basic, cystatin C is discharged retaining good correlation with creatinine which is unabsorbed at the renal tubule, regardless of the presence or absence of urinary proteins. Therefore, cystatin C has a physical property capable of reflecting the emergence of renal tubular protein urine most sensitively compared with α1-microglobulin and β2-microglobulin currently measured as an evaluation method of the renal tubular function.

From the above, the method for diagnosing the kidney disease by the cystatin C concentration and the creatinine concentration in urine is illustrated below. First, the cystatin C concentration and the creatinine concentration are measured in urine at any time in a large number of normal subjects (urinary protein is negative, and urinary tract infection is not observed) to calculate the cystatin C concentration/creatinine concentration ratio (hereinafter, referred to as "cystatin C/creatinine ratio" or "UCCR") in respective specimens. Then, using these data, a cut-off value of the cystatin C/creatinine ratio in urine of the normal subjects is calculated. This cystatin C/creatinine ratio is an index for diagnosing the presence or absence of renal tubular disorder in patients with diabetes. Additionally, cut-off values for the cystatin C concentration and the creatinine concentration in urine of normal subjects are also calculated. This cystatin C value is an index for diagnosing the presence or absence of glomerular hypertension in patients with diabetes in whom a renal tubular disorder is not observed. Also, the creatinine value is an index for diagnosing the presence or absence of glomerular hypertension in patients with diabetes in whom a renal tubular disorder is observed. With respect to any of the above indices, when an index obtained from a subject shows a value equal to or higher than the cut-off value obtained from a large number of normal volunteers, the subject is diagnosed to exhibit symptoms which the index indicates.

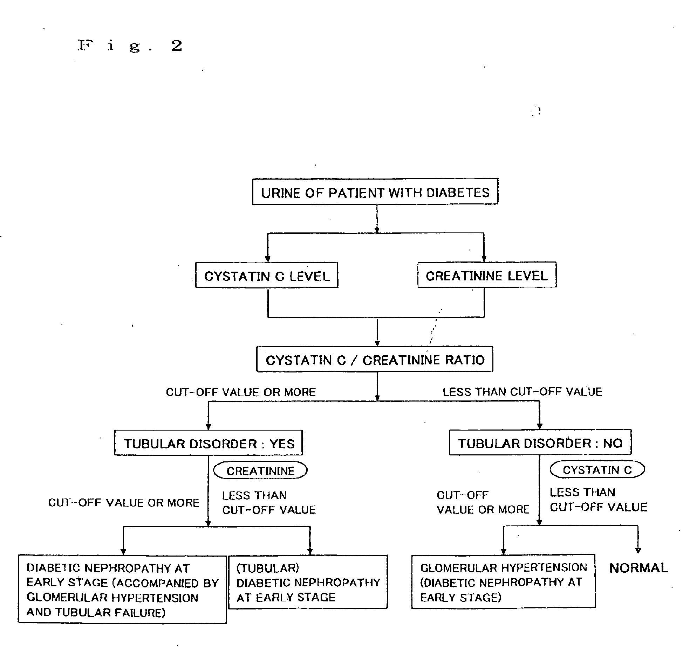

In the present invention, the diagnosis of kidney disease is carried out by appropriately combining three cut-off values. Fig. 2 shows procedures for early diagnosis of diabetic nephropathy. The cystatin C concentration and the creatinine concentration are measured in a patient with diabetes to calculate the cystatin C/creatinine ratio. When the cystatin C/creatinine ratio is equal to or higher than the cut-off value, the patient is diagnosed to have a renal tubular disorder. Next, with respect to this patient with diabetes in whom a renal tubular disorder has been observed, when the creatinine value shows a value equal to or higher than the cut-off value, the patient is diagnosed to exhibit glomerular hypertension in addition to the renal tubular disorder. Meanwhile, when the creatinine value shows a value equal to or lower than the cut-off value, the patient is diagnosed to be diabetic nephropathy exhibiting no glomerular hypertension and having the renal tubular disorder.

Subsequently, with respect to a patient with diabetes in whom the cystatin C/creatinine ratio shows a value equal to or lower than the cut-off value and no renal tubular disorder is observed, when the cystatin C value shows a value equal to or higher than the cut-off value, the patient is diagnosed to be diabetic nephropathy exhibiting the glomerular hypertension. Meanwhile, when the cystatin C value shows a value equal to or lower than the cut-off value, the patient is diagnosed not to be diabetic nephropathy exhibiting the glomerular hypertension.

As the above, it is possible to diagnose the renal tubular and glomerular disorders of the kidney disease in the patient with diabetes by measuring the cystatin C and creatinine in the urine.

### Description of the Embodiments

### (A) Measurement of urinary cystatin C by ELISA

The measurement of a urinary cystatin C value was carried out by an enzyme-linked immunosorbent assay (ELISA) using a 96-well microplate (Nunc-Immuno Plate: Poly Sorp). Rabbit polyclonal anti-human cystatin C antibody (DAKO) used for a solid phase was diluted at 5 µg/ml using 0.05 mol/L tris hydrochloric acid buffer (pH 8.4), and 100 µl of aliquot was dispensed in each well to absorb by reacting at 4°C for 24 hours. At measurement, excess antibody was washed out with deionized water, and 100 µl of tris buffer (0.1 mol/L, pH 8.0) containing 1% bovine serum albumin (BSA)(Bayer) and 0.3 mol/L disodium hydrogen phosphate was dispensed in each well as a blocking agent (1.05 µl of anti-human cystatin C is contained per well) .

Then, 50 µl of a urine sample was added and mixed, reacted at 37°C for one hour, and subsequently washed three times with deionized water containing 0.05% of Tween 20. Subsequently, 100 µl of alkali phosphatase labeled anti-human cystatin C antibody solution (tris buffer containing 1% BSA) was added to each well, and mixed, then reacted at 37°C for one hour, and washed three times as with the above.

Additionally, 100 µl of a substrate buffer of the Kind-King method (Mizuho Medy) was added to each well, and reacted at 37°C for 30 min.

Then, 100 µl of color reagent (Mizuho Medy) was added to each well to develop a color, and the color was compared at a wavelength of 510/630 nm using a colorimeter for a microplate (Sanko Junyaku). The concentration of urinary cystatin C was calculated from a standard curve of cystatin C standard solutions (Human Recombinant Cystatin C Calibrator, DAKO) simultaneously measured.

### (B) Measurement of urinary creatinine by Jaffe method

The urinary creatinine was measured by utilizing a Jaffe reaction which produces an orange red colored condensed matter by reacting creatinine with picric acid in an alkali solution. A deproteinizing agent (200 µl) (containing sodium tungstate phosphate) was added to 50 µl of urine diluted at 21 times with deionized water, stirred, left at room temperature for 10 min, and the supernatant was separated by centrifuge (2,500 rpm, 10 min). A picric acid solution (22 mmol/L, 50 µl) and 0.75 N sodium hydroxide solution (50 µl) were added to 100 µl of this supernatant, and left at 25°C for 20 min. Subsequently, the color was compared at a wavelength of 510 nm using a colorimeter for a microplate (Sanko Junyaku), and the concentration of urinary creatinine was calculated from a standard curve of creatinine standard solutions simultaneously measured.

### (C) Setting example of a cut-off value of the cystatin C concentration and the cystatin C/creatinine ratio in normal volunteers

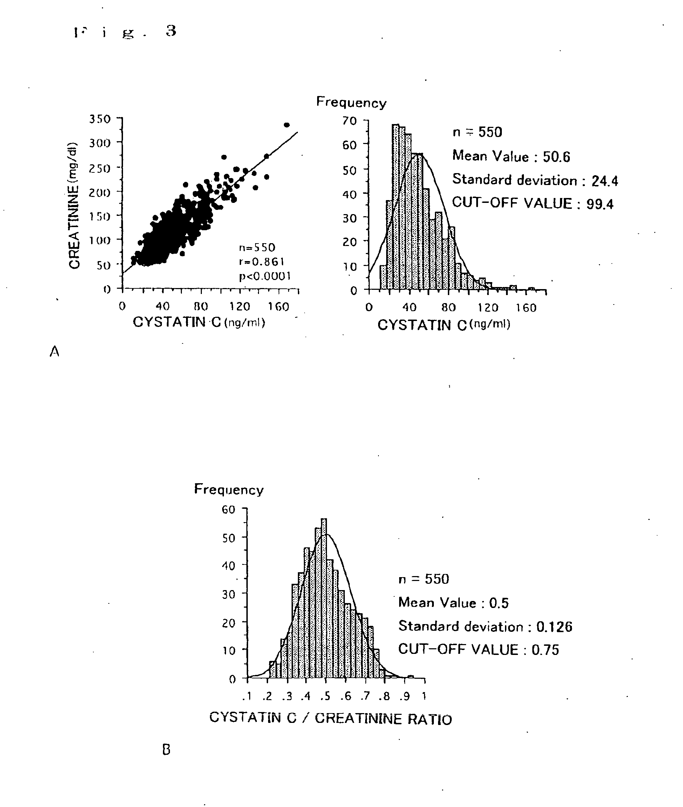

A setting example of a cut-off value of the urinary cystatin C concentration using 550 samples of normal urine is shown in Fig. 3A. A calculation example of a cut-off value of the cystatin C/creatinine ratio is shown in Fig. 3B.

### (D) Change of the cystatin C/creatinine ratio in cases of proteinuria and urinary tract infection

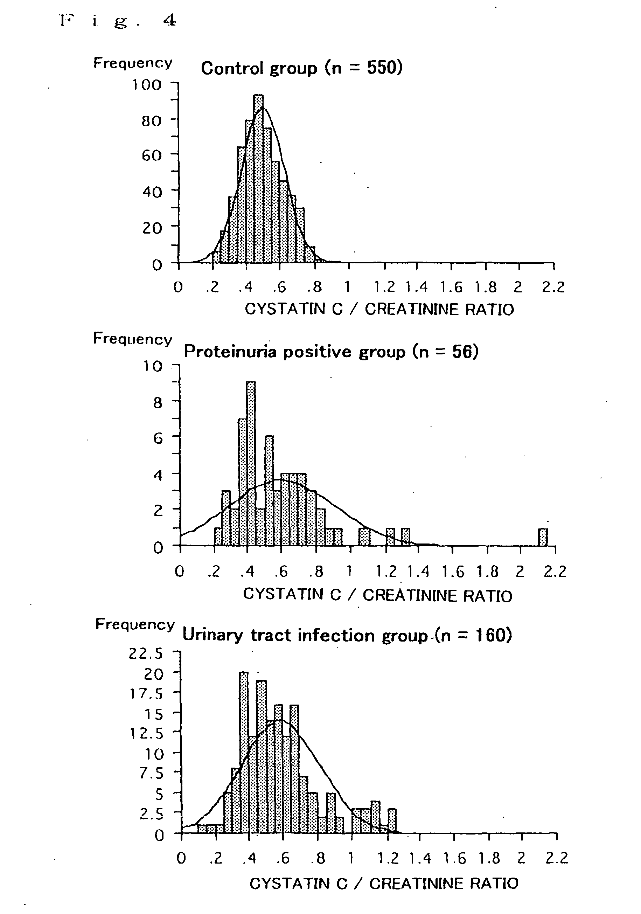

Changes of the cystatin C/creatinine ratio due to urinary proteins and infectious factors (example is shown in urinary tract infection) which are positively involved in the formation of tubulointerstitial lesions are shown in Fig. 4. Both factors are involved in the tubulointerstitial disorder, and the cystatin C/creatinine ratio showed relatively higher values along with the advancement of lesions, compared to the control group (normal volunteers).

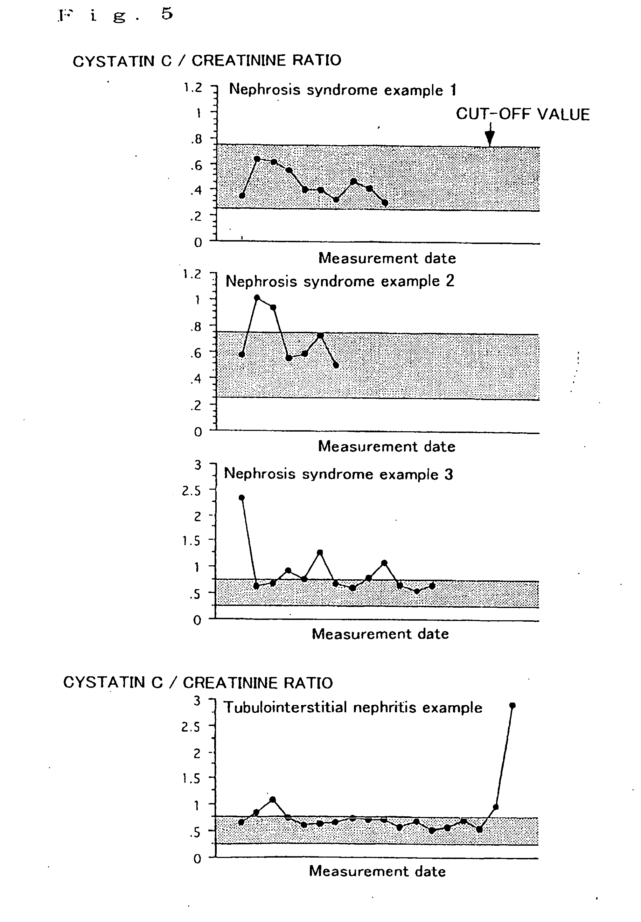

Examples of transitional changes of the cystatin C/creatinine ratio in patients with nephrosis exhibiting persistent proteinuria and a patient with tubulointerstitial nephritis are shown in Fig. 5.

### (E) Comparison of detection sensitivity of tubulointerstitial lesions involved in urinary tract infection.

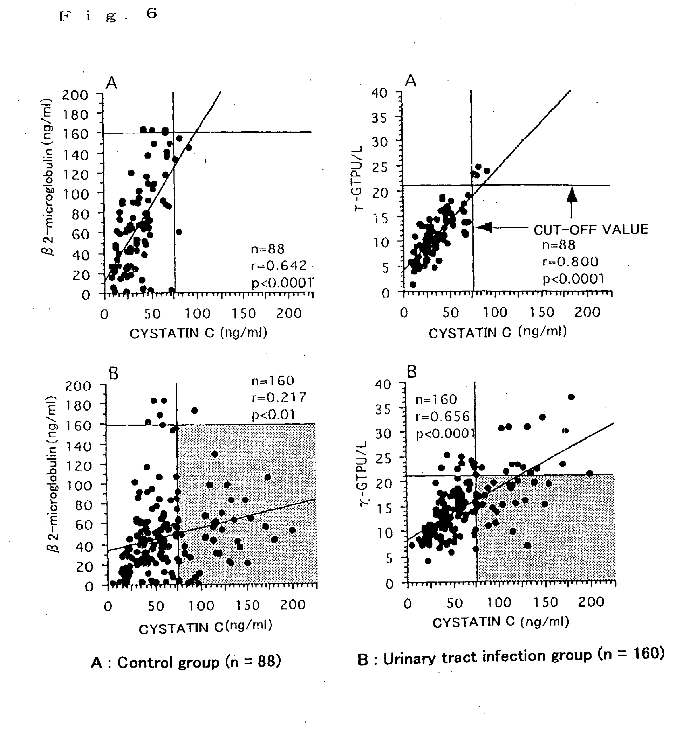

With respect to β2-microglobulin and γ-GTP which have been frequently used as markers for detecting the tubulointerstitial lesion and cystatin C which the present inventors have discovered, it has been confirmed that cystatin C is far superior in disease detection sensitivity than β2-microglobulin and γ-GTP as is shown in Fig. 6.

### (F) Setting examples of cut-off values of the cystatin C concentration, creatinine concentration and cystatin C/creatinine ratio in normal volunteers (normal in urinary protein, leukocytes and bacteria).

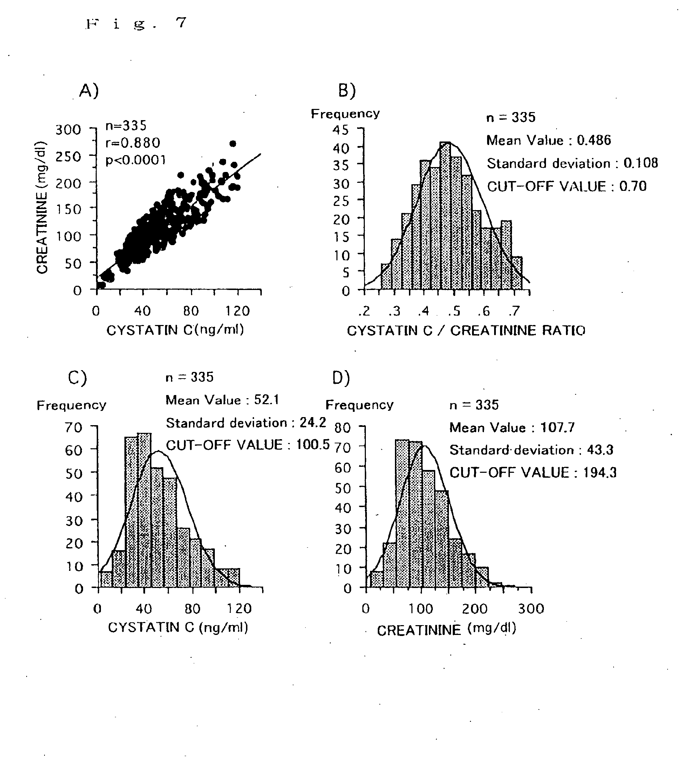

Fig. 7 shows calculation examples of the cut-off values of the cystatin C concentration, creatinine concentration and cystatin C/creatinine ratio (UCCR) in 335 samples of normal urine considered as being normal in renal tubular and glomerular functions. Fig. 7A shows the relation of the cystatin C concentration and the creatinine concentration in urine in the normal group. Fig. 7B shows the distribution and the cut-off value of the cystatin C/creatinine ratio in the normal group. According to Fig. 7B, the cut-off value is calculated as 0.70. Fig. 7C shows the distribution and the cut-off value of the urinary cystatin C concentration in the normal group, and according to Fig. 7C, the cut-off value is calculated as 100. 5. Fig. 7D shows the distribution and the cut-off value of the urinary creatinine concentration in the normal group, and according to Fig. 7D, the cut-off value is calculated as 194.3.

### (G) Early diagnosis examples of diabetic nephropathy for patients with diabetes

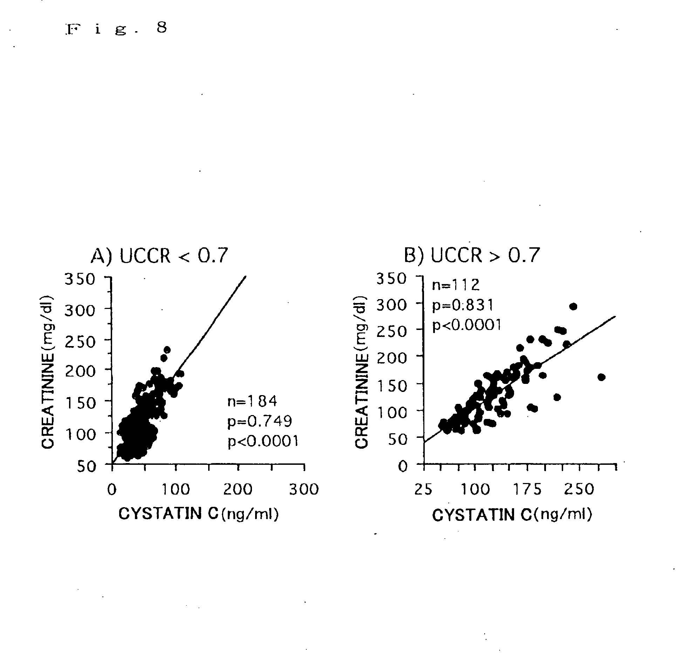

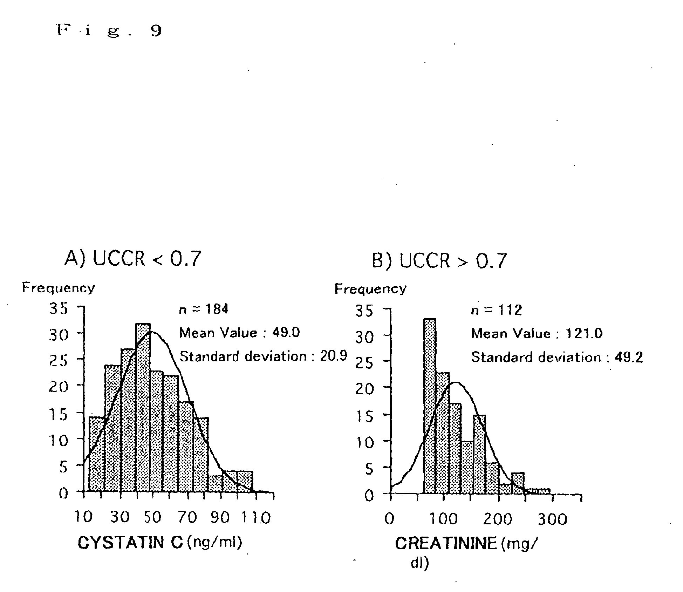

Examples of early discovery of diabetic nephropathy are shown using 296 samples of urine from patients diagnosed as diabetic.
(1) The cystatin C concentration and the creatinine concentration were measured for 296 samples of urine from patients with diabetes.
(2) The cystatin C/creatinine ratio (UCCR) in each individual was calculated.
(3) The diagnosis was performed according to the early discovery procedure of diabetic nephropathy in Fig. 2.
(4) First, the presence or absence of a tubulointerstitial disorder was determined by the individual UCCR value of the patient with diabetes using the cut-off value (0.70) of the cystatin C/creatinine ratio (UCCR) previously obtained from the normal group as the standard value. As is shown in Fig. 8A, the UCCR values are 0.70 or less in 184 cases, and these were determined not to have the tubulointerstitial disorder. Next, for these 184 cases, whether there is a finding of glomerular hypertension or not was determined using the cut-off value (100.5) of the cystatin C concentration obtained from the normal group as the standard value. From Fig. 9A, 3 in 184 cases were determined to be glomerular hypertension, and 181 cases were determined to have not yet developed nephropathy.
(5) Subsequently, for 112 cases determined to have the tubulointerstitial disorder (Fig. 8B), the determination whether exhibiting the glomerular hypertension or not was carried out. This determination was carried out using the cut-off value (194.3) of the creatinine concentration obtained from the normal group in Fig. 7D. As a result, as shown in Fig. 9B, 8 in 112 cases were determined to have complications of glomerular hypertension.
(6) Collectively showing the above determination results, according to the early discovery procedure of the diabetic nephropathy, in 296 patients with diabetes, 181 cases had not yet developed nephropathy, 3 cases correspond to the glomerular hypertension state, 104 cases correspond to the tubulointerstitial disorder, and 8 cases were determined to have complications of glomerular hypertension and tubulointerstitial disorder.

### (E) Problematic points of the conventional method (β2-microglobulin measurement method) in the early diagnosis for patients with diabetes exhibiting pyuria

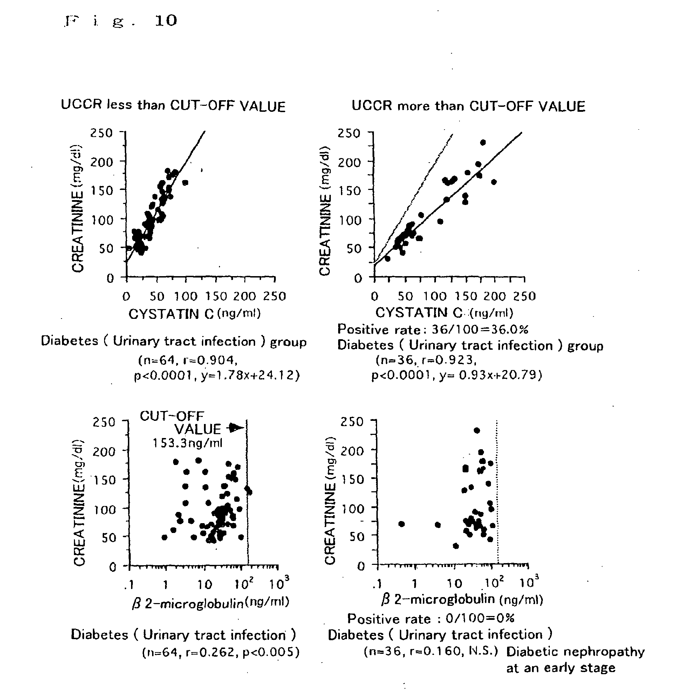

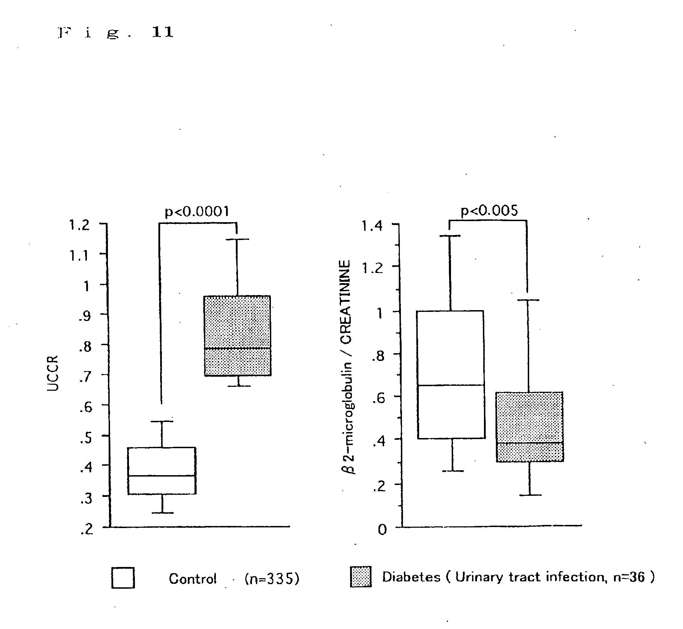

Usefulness for early diagnosis of nephropathy by the urinary cystatin C/creatinine ratio method and by conventionally utilized β2-microglobulin method was compared using 100 samples of urine of patients with diabetes combining urinary tract infection. As is shown in Fig. 10, abnormality was observed in 36 of 100 cases (36%) in the cystatin C/creatinine ratio method whereas no abnormality was detected in the β2-microglobulin method. Fig. 11 shows comparative data of distributions of the urinary cystatin C/creatinine ratio and the β2-microglobulin/creatinine ratio from urine in the same cases. From these comparative data, it has been found that renal disorder cannot be detected by β2-microglobulin of the conventional method because β2-microglobulin is degraded by protease mainly derived from leukocytes in urine of patients with urinary tract infection.

### (F) Diagnostic examples of tubulointerstitial disorder following glomerular lesion

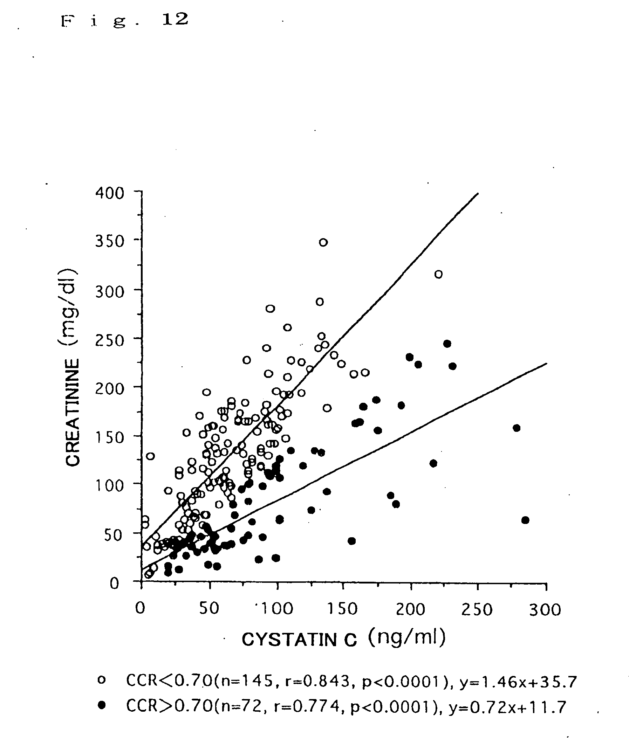

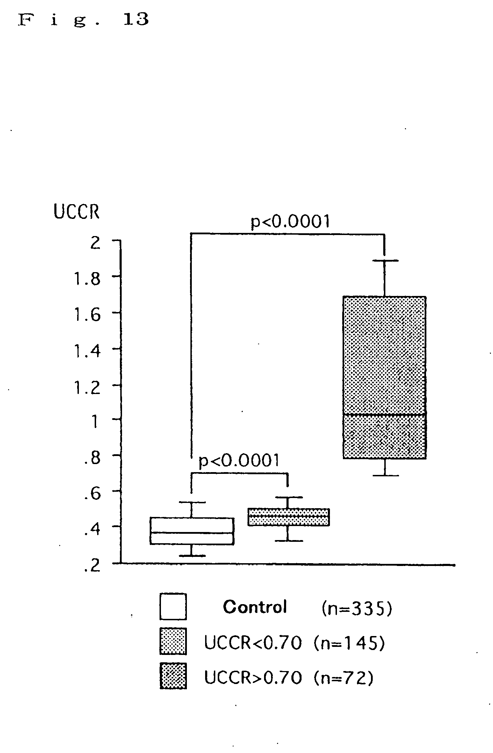

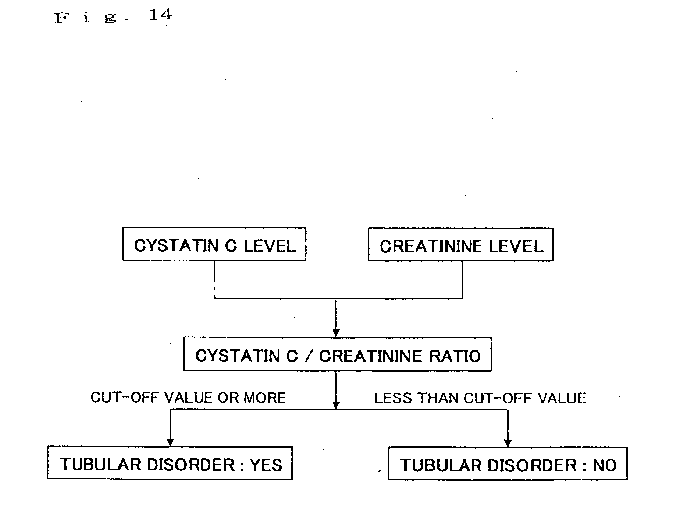

Since it has been shown that urinary protein is a risk factor of the tubulointerstitial disorder, the correlation of the urinary cystatin C and the urinary creatinine was examined concerning a higher group and a lower group than the cut-off value (0.70) of the urinary cystatin C/creatinine ratio for 217 cases (protein concentration=96.6 mg/dl ± 64.2 mg/dl, mean value ± standard deviation) of urine from patients exhibiting proteinuria. As is shown in Fig. 12, in the lower group than the cut-off value, a regression formula was approximated to a regression formula in the normal group, but in the higher group than the cut-off value, only cystatin C was changed to higher values, and a regression formula was significantly changed compared to that in the normal group. The comparison of the UCCR in the normal group and those in the proteinuria group with UCCR>0. 70 was as Fig. 13. In this way, the diagnostic procedure of the tubulointerstitial disorder following the glomerular lesion was shown in Fig. 14.

### Industrial Applicability

As described in detail above, according to the kit for diagnosing kidney diseases of the present invention, it is possible to make an early diagnose of the presence or absence of a glomerular lesion and tubulointerstitial disorder by especially measuring urinary cystatin C and creatinine of patients with diabetes in combination.

## Claims

1. A method for diagnosis, wherein kidney diseases are diagnosed by measuring a cystatin C concentration and a creatinine concentration discharged in urine of a patient with diabetes, wherein a ratio of the cystatin C concentration to the creatinine concentration (UCCR) is calculated, wherein said ratio is compared to a first standard value being a cut-off value obtained by measuring at any time in a large number of normal volunteers a cystatin C concentration and a creatinine concentration discharged in urine and calculating a ratio of the cystatin C concentration to the creatinine concentration (UCCR),
wherein it is determined whether said ratio exceeds said first standard value; and wherein the patient is diagnosed to exhibit renal tubular disorder if said ratio is determined to exceed said first standard value.

2. The method for diagnosis according to Claim 1, wherein an immunoassay means such as an enzyme immunoassay method, a latex agglutination reaction and an immunochromatography method is used for the measurement of cystatin C concentration, and a chemical method or an enzymatic method is used for the measurement of creatinine concentration.

3. The method for diagnosis according to claim 2, wherein at least an anti-human cystatin C antibody and a reagent for the measurement of creatinine concentration by a chemical or an enzymatic method are used.

4. The method for diagnosis according to any one of claims 1 to 3,
wherein,for diagnosing the presence or absence of glomerular hypertension in said patients in whom a renal tubular disorder is not diagnosed, said cystatin C concentration is compared with a second standard value being a cut-off value calculated from measurements of cystatin C concentration discharged in urine in a large number of normal subjects,
wherein it is determined whether said cystatin C concentration exceeds said second standard value;
and wherein the presence or absence of glomerular hypertension in patients in whom a renal tubular disorder is not diagnosed is diagnosed if said cystatin C concentration is determined to exceed said second standard value.

5. The method for diagnosis according to any one of claims 1 to 3,
wherein, for diagnosing the presence or absence of glomerular hypertension in said patients in whom a renal tubular disorder is diagnosed,
said creatinine concentration is compared with a third standard value being a cut-off value calculated from measurements of creatinine concentration discharged in urine in a large number of normal subjects,
wherein it is determined whether said creatinine concentration exceeds said third standard value;
and wherein the presence or absence of glomerular hypertension in patients in whom a renal tubular disorder is diagnosed is diagnosed if said creatinine concentration is determined to exceed said third standard value.

## Patentansprüche

1. Eine Methode zur Diagnose, bei der Nierenerkrankungen diagnostiziert werden durch Messen der Konzentration von Cystatin C und der Konzentration von Creatinin nach Ausscheidung im Urin von einem Patienten mit Diabetes, wobei ein Verhältnis der Cystatin C Konzentration zur Creatinin Konzentration (UCCR) errechnet wird, wobei dieses Verhältnis verglichen wird mit einem ersten Standardwert, der ein "cut-off" Wert ist, der durch die Messung der Konzentration von Cystatin C und der Konzentration von Creatinin nach Ausscheidung im Urin in einer großen Anzahl von normalen Freiwilligen zu einer beliebigen Zeit und durch die Errechnung des Verhältnisses der Cystatin C Konzentration zur Creatinin Konzentration (UCCR) ermittelt wird, wobei bestimmt wird, ob das genannte Verhältnis den genannten ersten Standardwert übersteigt; und wobei bei einem Patienten diagnostiziert wird, daß er renale Tubulus-Funktionsstörungen zeigt, falls bei dem genannten Verhältnis ermittelt wird, dass der besagte erste Standardwert überschritten wird.

2. Die Methode zur Diagnose nach Anspruch 1, wobei ein Immunoassay-Mittel wie eine Enzymimmunoassay Methode, eine Latexagglutinationsreaktion und eine Immunochromatographie Methode benutzt wird für die Messung der Cystatin C Konzentration und eine chemische Methode oder eine enzymatische Methode für die Messung der Creatinin Konzentration.

3. Die Methode zur Diagnose nach Anspruch 2, wobei wenigstens ein Anti-human Cystatin C Antikörper und ein Reagenz für die Messung der Creatinin Konzentration mit einer chemischen oder einer enzymatischen Methode benutzt wird.

4. Die Methode zur Diagnose nach irgendeinem der Ansprüche 1 bis 3, wobei zur Diagnostizierung des Vorhandenseins oder der Abwesenheit glomerulären Hochdruckes bei den genannten Patienten, bei denen eine renale Tubulus-Funktionsstörung nicht diagnostiziert wurde, die genannte Cystatin C Konzentration verglichen wird mit einem zweiten Standardwert, der ein "cut-off" Wert ist, der durch die Messung der Cystatin C Konzentration nach Ausscheidung im Urin in einer grossen Anzahl von normalen Personen ermittelt wird, wobei bestimmt wird, ob die genannte Cystatin C Konzentration den genannten zweiten Standardwert übersteigt; und wobei das Vorhandensein oder die Abwesenheit glomerulären Hochdrucks bei Patienten, bei denen eine renale Tubulus-Funktionsstörung nicht diagnostiziert wurde, falls die genannte Cystatin C Konzentration so bestimmt wurde, dass sie den genannten zweiten Standardwert übersteigt.

5. Die Methode zur Diagnose nach irgendeinem der Ansprüche nach 1 bis 3, wobei zur Diagnostizierung des Vorhandenseins oder der Abwesenheit glomerulären Hochdruckes bei den genannten Patienten, bei denen eine renaleTubulus-Funktionsstörung diagnostiziert wurde, die genannte Creatinin Konzentration verglichen wird mit einem dritten Standardwert, der ein "cut-off" Wert ist, der durch die Messung der Creatinin Konzentration nach Ausscheidung im Urin in einer grossen Anzahl von normalen Personen ermittelt wird, wobei bestimmt wird, ob die genannte Creatinin Konzentration den genannten dritten Standardwert übersteigt; und wobei das Vorhandensein oder die Abwesenheit glomerulären Hochdruckes bei Patienten, bei denen eine renale Tubulus-Funktionsstörung diagnostiziert wird, diagnostiziert wird, falls bei der genannten Creatinin Konzentration ermittelt wurde, dass sie den genannten dritten Standardwert übersteigt.

## Revendications

1. Procédé de diagnostic, dans lequel des maladies du rein sont diagnostiquées en mesurant une concentration de cystatine C et une concentration de créatinine évacuée dans l'urine d'un patient souffrant de diabète, dans lequel est calculé un rapport entre la concentration de cystatine C et la concentration de créatinine (UCCR), dans lequel ledit rapport est comparé à une valeur standard qui est une valeur de délimitation obtenue en mesurant à tout moment parmi un grand nombre de volontaires normaux une concentration de cystatine C et une concentration de créatinine évacuée dans l'urine et en calculant un rapport entre la concentration de cystatine C et la concentration de créatinine (UCCR), dans lequel il est déterminé si ledit rapport excède ladite première valeur standard; et
dans lequel il est diagnostiqué que le patient présente un trouble tubulaire rénal s'il est déterminé que ledit rapport excède ladite première valeur standard.

2. Procédé de diagnostic selon la revendication 1, dans lequel est utilisé un moyen d'immuno-essai, tel qu'un procédé d'immuno-essai enzymatique, une réaction d'agglutination de latex et un procédé d'immuno-chromatographie, pour la mesure de la concentration de cystatine C, et il est utilisé un procédé chimique ou un procédé enzymatique pour la mesure de la concentration de créatinine.

3. Procédé de diagnostic selon la revendication 2, dans lequel il est utilisé au moins un anticorps cystatine C anti-humain et un réactif pour la mesure de la concentration de créatinine par un procédé chimique ou enzymatique.

4. Procédé de diagnostic selon l'une quelconque des revendications 1 à 3, dans lequel, pour diagnostiquer la présence ou l'absence d'hypertension glomérulaire dans lesdits patients dans lesquels il n'est pas diagnostiqué de trouble tubulaire rénal, ladite concentration de cystatine C est comparée à une deuxième valeur standard qui est une valeur de délimitation calculée à partir de mesures de concentration de cystatine C évacuée dans l'urine dans un grand nombre de sujets normaux,
dans lequel il est déterminé si ladite concentration de cystatine C excède ladite deuxième valeur standard;
et dans lequel est diagnostiquée la présence ou l'absence d'hypertension glomérulaire dans des patients dans lesquels il n'est pas diagnostiqué de trouble tubulaire rénal s'il est déterminé que ladite concentration de cystatine C excède ladite deuxième valeur standard.

5. Procédé de diagnostic selon l'une quelconque des revendications 1 à 3, dans lequel, pour diagnostiquer la présence ou l'absence d'hypertension glomérulaire dans lesdits patients dans lesquels il est diagnostiqué un trouble tubulaire rénal, ladite concentration de créatinine est comparée à une troisième valeur standard qui est une valeur de délimitation calculée à partir de mesures de concentration de créatinine évacuée dans l'urine dans un grand nombre de sujets normaux, dans lequel il est déterminé si ladite concentration de créatinine excède ladite troisième valeur standard,
et dans lequel est diagnostiquée la présence ou l'absence d'hypertension glomérulaire dans des patients dans lesquels il est diagnostiqué un trouble tubulaire rénal s'il est déterminé que ladite concentration de créatinine excède ladite troisième valeur standard.
